(19) Europäisches Patentamt
European Patent Office
Office européen des brevets

(11) Publication number: 0 375 319 A2

(12) # EUROPEAN PATENT APPLICATION

(21) Application number: 89313200.1

(22) Date of filing: 18.12.89

(51) Int. Cl.5: A61K 37/64, A61K 31/38

(30) Priority: 19.12.88 US 286004

(43) Date of publication of application: 27.06.90 Bulletin 90/26

(84) Designated Contracting States: AT BE CH DE ES FR GB IT LI LU NL SE

(71) Applicant: MERCK & CO. INC.
126, East Lincoln Avenue P.O. Box 2000
Rahway New Jersey 07065-0900(US)

(72) Inventor: Patchett, Arthur A.
1090 Minisink Way
Westfield, NJ 07090(US)
Inventor: Lotti, Victor J.
214 Brookside Circle
Harleysville, PA 19438(US)
Inventor: Baldwin, John J.
621 Gypey Hill Circle
Gwynedd Valley, PA 19437(US)

(74) Representative: Hesketh, Alan, Dr. et al
European Patent Department Merck & Co., Inc. Terlings Park Eastwick Road
Harlow Essex, CM20 2QR(GB)

(54) **Angiotensin converting enzyme inhibitors in the treatment of glaucoma.**

(57) Angiotensin converting enzyme inhibitors with a lactam ring of 7-9 members and bicyclic analogs either alone or in combination with carbonic anhydrase inhibitors are useful in the treatment of glaucoma and ocular hypertension associated therewith.

EP 0 375 319 A2

## ANGIOTENSIN CONVERTING ENZYME INHIBITORS IN THE TREATMENT OF GLAUCOMA.

### SUMMARY OF THE INVENTION

This invention is concerned with the use of angiotensin converting enzyme (ACE) inhibitors of structural formula:

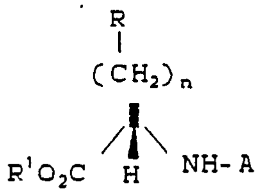

I

wherein A is a 7-9 membered lactam and R and $R^1$ are as hereinafter defined, in the treatment of glaucoma and ocular hypertension associated therewith.

The invention is also concerned with ophthalmological formulations comprising at least one of the ACE inhibitors as active ingredient.

The invention is also concerned with the use of the ACE inhibitors and ophthalmological formulations thereof in combination with topically effective carbonic anhydrase (CA) inhibitors.

### BACKGROUND OF THE INVENTION

Glaucoma is an ocular disease complex associated with an elevated pressure within the eye (i.e., ocular hypertension, intraocular pressure, IOP). As a result of the elevated IOP, damage to the optic nerve head resulting in irreversible loss of visual function may ensue. Untreated, this condition may eventually lead to blindness.

Ocular hypertension, i.e., the condition of elevated intraocular pressure without optic nerve head damage or characteristic glaucomatous visual field loss, is now believed by the majority of ophthalmologists to represent the earliest phase in the onset of glaucoma.

A number of the drugs presently employed to treat glaucoma are not entirely satisfactory, particularly in the earliest course of the disease when the side effects they produce are often worse than the symptoms of the disease.

Epinephrine used as a topical solution, must be utilized cautiously in patients with high blood pressure, diabetes, hyperthyroidism and cerebral artereosclerosis due to the possibility of systemic action.

Timolol, a clinically utilized, topically applied agent for lowering intraocular pressure, must be used with caution in patients in whom beta-adrenergic blockade may be undesirable. Systemic absorption of topically administered timolol and systemic beta blockade are responsible for the contraindication of timolol therapy for glaucoma in patients with compromised pulmonary function.

Pilocarpine, a topical drug, although considered systemically harmless and quite effective, may cause considerable local difficulties. Pupil constriction may cause the eye to lose its ability to adapt from light to dark. Accomodation may become stimulated so that the patient's refraction is sometimes incorrect and vision becomes blurred. The drug itself may cause a local vasodilation and red eyes. Irritation is common.

Carbonic anhydrase inhibitors have been used systemically but they have a number of disadvantages. While effective in lowering intraocular pressure, they often cause a numbness and tingling, gastrointestinal upsets and, frequently, depression, lethargy, a loss of appetite, and general malaise.

Many of the problems associated with Systemic CA inhibitors are overcome by the use of topically effective CA inhibitors.

With the present invention there is provided a method of treating glaucoma and ocular hypertension by the topical ocular administration of a formulation comprising as an active ingredient an ACE inhibitor of formula I either alone or in combination with a topically active CA inhibitor.

The ACE inhibitors useful in the novel formulations and method of treatment of this invention in part are described in U.S. Patents 4,587,050; 4,587,238; 4,617,301; 4,629,787; 4,661,479; and 4,680,392 the

disclosures of which are incorporated herein by reference.

Other ACE inhibitors useful in the novel method of treatment of this invention are: CS-622, described in Biochemical and Biophysical Research Communications, 152, 457 (1988); CV-5975 described in Japan, J. Pharmacol., 47, 135-141 (1988); CGS-16617 described in Arch. Int. Pharmacolyn., 292, 266-280 (1988); Cilazapril (RO-312848); CGS-14824A; and those described in J. Med. Chem., 28, 1603-1606 (1985); J. Med. Chem., 31, 422-428 (1988); and J. Am. Chem. Soc., 109, 7914-7915 (1987).

## DETAILED DESCRIPTION OF THE INVENTION

The novel pharmaceutical formulation of this invention is a topical ophthalmologically acceptable composition for the treatment of glaucoma and ocular hypertension associated therewith which comprises an effective ocular antihypertensive amount of an ACE inhibitor, either alone or in combination with a topically effective CA inhibitor, and an ophthalmologically acceptable carrier, wherein the ACE inhibitor has structural formula I:

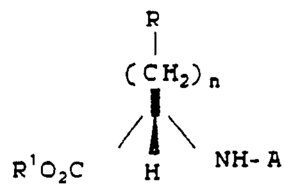

I

or an ophthalmologically acceptable salt thereof, wherein:

R is

1) hydrogen,

2) phenyl,either unsubstituted or substituted with hydroxy, $C_{1-3}$alkoy, $C_{1-3}$alkyl, amino-$C_{1-3}$ alkyl, mono-($C_{1-3}$alkyl)amino-$C_{1-3}$alkyl, or di($C_{1-3}$alkyl)amino-$C_{1-3}$alkyl,

3) 1-naphthyl,

4) 4-piperidinyl,

5) pyridyl,

6) quinolinyl,

7) thiazolyl, or

8) amino either unsubstituted or substituted with one or two $C_{1-3}$alkyl groups; n is 1 to 5;

$R^1$ is

1) H,

2) $C_{1-6}$alkyl, or

3) phenyl-$C_{1-3}$alkyl; A is

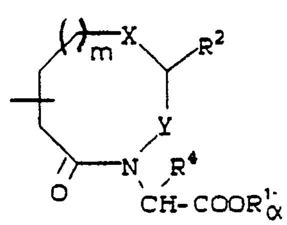

wherein:

m is 0, 1 or 2;

X is

1)

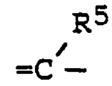,

2) $-CH_2-$
3) -S-
4) -SO-, or
5) $-SO_2-$; Y is $CHR^3$ or $N-R^3$;

$R^1{}_\alpha$ is
1) H,
2) $C_{1-6}$alkyl; or
3) phenyl-$C_{1-3}$alkyl; $R^2$ is
1) H,
2) $C_{1-3}$alkyl,
3) 2-thienyl, or
4) phenyl; $R^4$ is
1) hydrogen, or
2) $C_{1-3}$alkyl; $R^3$ and $R^5$ are hydrogen, or

$R^2$ and $R^3$, or $R^2$ and $R^5$ taken together with the atoms to which they are attached, represent:

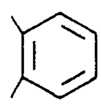 ; or

$R^3$ and $R^4$ taken together with the atoms to which they are attached represent:

1)

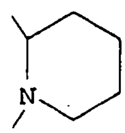

2)

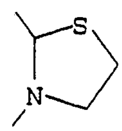 or

3)

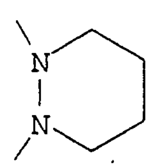.

The novel method of treatment of this invention comprises the topical ocular administration of an effective ocular antihypertensive amount of an ACE inhibitor either alone or in combination with a topically effective CA inhibitor to a patient in need of such treatment wherein the ACE inhibitor is of structural formula I.

In the ACE inhibitors useful in the novel formulations and method of treatment, it is preferred that R is phenyl, n is 2, $R'$ is H, m is 0, and X is $-CH_2-$. It is also preferred that if Y is $-CHR^3$, $R^3$ and $R^4$ taken together with the atoms to which they are attached represent

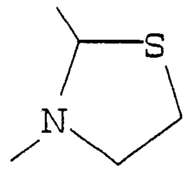

Preferred species of the ACE inhibitors useful in the novel formulation and method of treatment of this invention include the following wherein the sterochemistry in the acyclic moiety is as in natural amino acids and as shown in Stucture I:

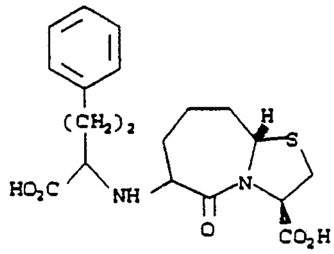

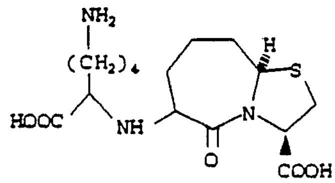

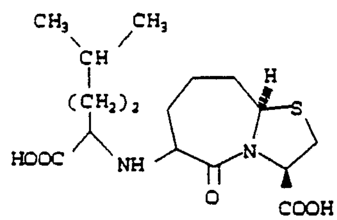

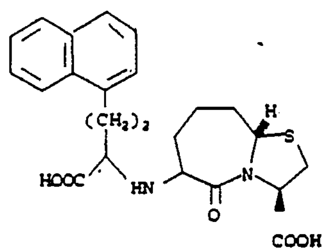

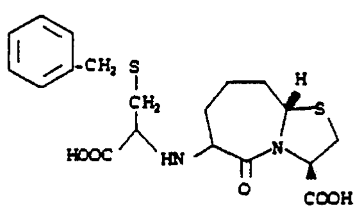

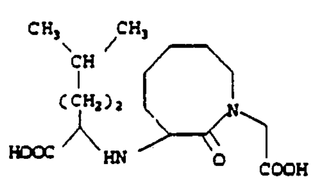

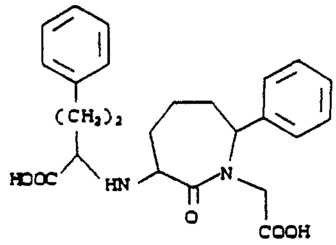

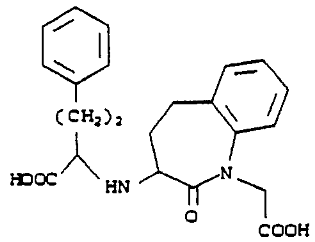

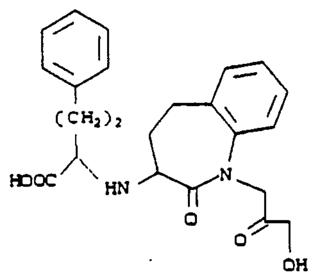

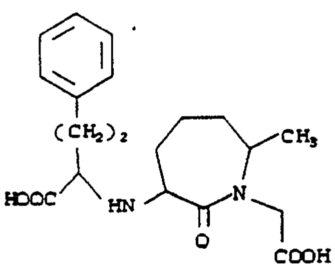

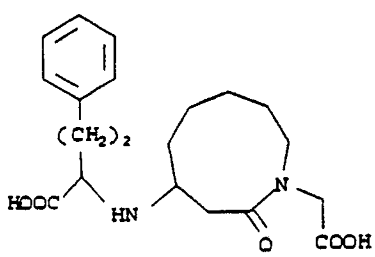

The active compounds of the invention (ACE inhibitors) are administered in the form of ophthalmic pharmaceutical compositions adapted for topical administration to the eye; such as solutions, suspensions, ointments and solid inserts. Formulations of these compounds may contain from 0.01 to 5% and especially 0.25% to 2% of medicament. Other concentrations may be employed provided the dose is effective in lowering intraocular pressure. As a unit dosage form, between 0.01 to 2.5 mg., preferably 0.05 to 2.5 mg., and especially 0.1 to 1.0 mg. of the active compound is applied to the human eye, generally on a daily basis, preferably in divided doses. Individual dosage requirements are variable, however, and must be

administered on the basis of the severity of the disease and the response of the patient.

To prepare suitable dosage forms, the active compounds may be conveniently admixed with a non-toxic pharmaceutically acceptable carrier suitable for topical ophthalmologic administration. Typical of such pharmaceutically acceptable carriers are, for example, water, mixtures of water and water miscible solvents such as lower alkanols or vegetable oils, petroleum based jelly, or including also from 0.5 to 5% by weight of water soluble polymers such as cellulose derivatives such as methyl cellulose, alkali metal carboxymethyl cellulose, hydroxyethyl cellulose, hydroxypropyl cellulose, hydroxypropyl methyl cellulose; acrylates such as polyacrylic acids salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone polyvinyl methyl ether, polyethylene oxide, neutralized carbopol and gums such as gellan, rhamson and xanthan gum and mixtures of these polymers. The pharmaceutical preparation may also contain non toxic auxiliary substances such as emulsifying, preserving, wetting, bodying agents and the like, as for example, polyethylene glycols, carbowaxes, antibacterial preservative components commonly employed in ophthalmic formulations; buffering ingredients; and other conventional ingredients.

The pharmaceutical preparation may also be in the form of a solid insert. For example, one may use a solid water soluble polymer as the carrier for the medicament. Inserts that are known in the art that are suitable for this use include those described in British Patent 15611, and in United States Patents 3,993,071; 3,986,510; 3,868,445; and 3,867,510. Solid water insoluble inserts, such as those prepared from ethylene vinyl acetate copolymer, may also be utilized.

The compositions of the invention may include additional therapeutic agents in addition to the ACE inhibitor. For example antibiotics, autiinflammatory steroids, and anesthetics as well as other IOP lowering agents such as a carbonic anhydrase inhibitor may be present.

The carbonic anhydrase (CA) inhibitors useful in combination with the above described ACE inhibitors are aromatic or heteroaromatic sulfonamides capable of reducing elevated intraocular pressure following topical ocular administration

Such CA inhibitors are described in U.S. Patents 4,619,939; 4,677,115; 4,668,697; U.S Serial No. 191,085 (filed May 4, 1988); U.S. Serial No. 067,326 (filed June 26, 1987) corresponding to EP 296,879; and U.S. Serial No. 059,084 (filed June 8, 1987) corresponding to EP 295,049; the disclosures of which are incorporated herein by reference and have generic structural formula:

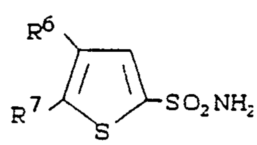

or an ophthalmologically acceptable salt thereof, wherein:

$R^6$ is hydrogen;

$R^7$ is

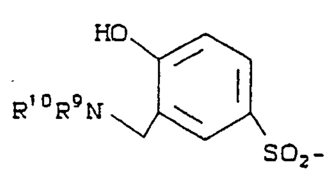

; or

$R^6$ and $R^7$ taken together with the carbons to which they are attached represent:

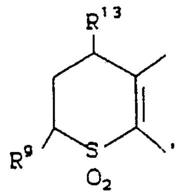

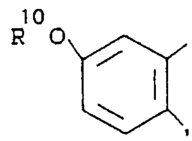 or

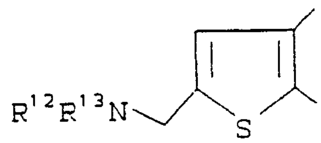

wherein $R^{13}$ is -OH or -$NHR^8$;
$R^8$ and $R^9$ are independently hydrogen or $C_{1-5}$alkyl;
$R^{10}$ is hydrogen or $C_{2-5}$alkanoyl;
$R^{11}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.

Preferred compounds within the genus have structural formulae:

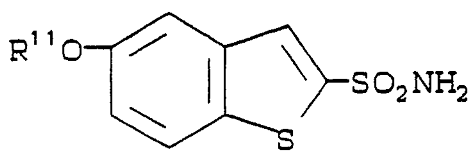

wherein $R^{11}$ is hydrogen or acetyl,

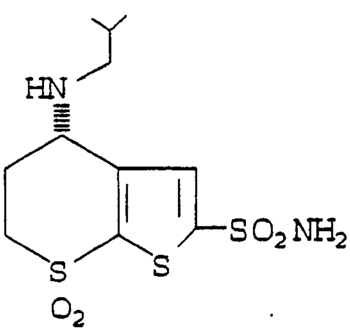 ,

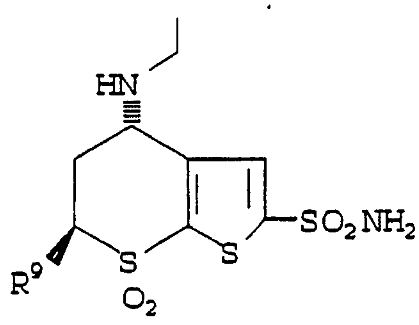

wherein $R^9$ is -$CH_3$ or -$C_2H_5$;

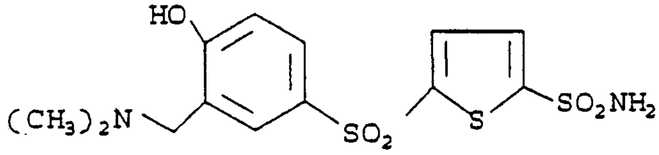,

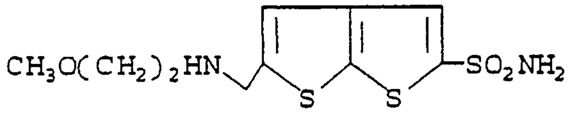

or

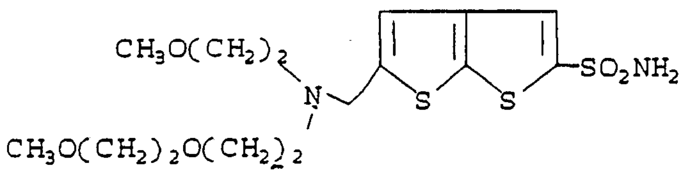.

If combined with a CA inhibitor in a novel formulation of this invention, the ACE inhibitor is used in a concentration of about one half that employed if it were the sole active ingredient, and the CA inhibitor is used in a pharmacologically equivalent concentration. In other words each of the ACE and CA inhibitors in the ophthalmic formulation is present at a concentration of about 0.005 to 2.5% and especially about 0.125 to 1%. As a unit dosage form 0.005 to 1.25 mg., preferably 0.025 to 1.25 mg., and especially 0.05 to 0.5 mg. of each active ingredient is applied to the human eye, generally on a daily basis and preferably in divided doses.

| EXAMPLE 1 | | |
|---|---|---|
| Formulations | | |
| ACE Inhibitor | 1 mg. | 15 mg. |
| Monobasic Sodium phosphate $\cdot 2H_2O$ | 9.38 mg. | 6.10 mg. |
| Dibasic Sodium phosphate $\cdot 12H_2O$ | 28.48 mg. | 16.80 mg. |
| Benzalkonium chloride | 0.10 mg. | 0.10 mg. |
| Water for injection q.s. ad. | 1.0 mg. | 1.0 ml. |

| EXAMPLE 2 | | |
|---|---|---|
| Formulations | | |
| ACE Inhibitor | 0.5 mg. | 7.5 mg. |
| CA Inhibitor | 0.5 mg. | 7.5 mg. |
| Monobasic Sodium phosphate $\cdot 2H_2O$ | 9.38 mg. | 6.10 mg. |
| Dibasic Sodium phosphate $\cdot 12H_2O$ | 28.48 mg. | 16.80 mg. |
| Benzalkonium chloride | 0.10 mg. | 0.10 mg. |
| Water for injection q.s. ad. | 1.0 ml. | 1.0 ml. |

The active ingredient(S), phosphate buffer salts and benzalkonium chloride are added to and dissolved in water. The pH of the composition is adjusted to 5-6 and diluted to volume. The composition is rendered sterile by exposure to ionizing radiation.

## Claims

1. An ophthalmic formulation for the treatment of glaucoma and ocular hypertension associated therewith which comprises an ophthalmologically acceptable carrier and 0.01 to 5% by weight of an ACE inhibitor of formula I

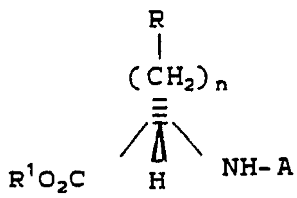

I

or an ophthalmologically acceptable salt thereof, wherein
R is
1) hydrogen,
2) phenyl,either unsubstituted or substituted with hydroxy, $C_{1-3}$alkoy, $C_{1-3}$alkyl, amino-$C_{1-3}$ alkyl, mono-($C_{1-3}$alkyl)amino-$C_{1-3}$alkyl, or di($C_{1-3}$a1ky1)amino-$C_{1-3}$alkyl,
3) 1-naphthyl,
4) 4-piperidinyl,
5) pyridyl,
6) quinolinyl,
7) thiazolyl, or
8) amino either unsubstituted or substituted with one or two $C_{1-3}$alkyl groups; n is 1 to 5;
$R^1$ is
1) H,
2) $C_{1-6}$alkyl; or
3) phenyl-$C_{1-3}$alkyl A is

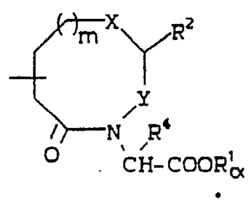

wherein:
m is 0, 1 or 2;
X is
1)

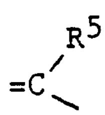 ,

2) $-CH_2-$
3) -S-
4) -SO-, or
5) $-SO_2-$; Y is $CHR^3$ or $N-R^3$;
$R^1_\alpha$is
1) H,
2) $C_{1-6}$alkyl; or
3) phenyl-$C_{1-3}$alkyl; $R^2$ is
1) H,

2) $C_{1-3}$alkyl,
3) 2-thienyl, or
4) phenyl; $R^4$ is
1) hydrogen, or
2) $C_{1-3}$alkyl; $R^3$ and $R^5$ are hydrogen, or

$R^2$ and $R^3$, or $R^2$ and $R^5$ taken together with the atoms to which they are attached, represent:

; or

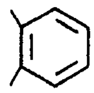

.

$R^3$ and $R^4$ taken together with the atoms to which they are attached represent:

1) 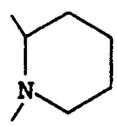

or

2) 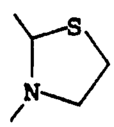

3) 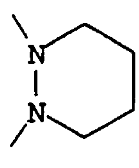 .

2. The formulation of Claim 1 wherein the ACE inhibitor is selected from:

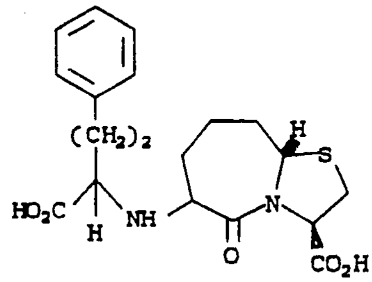

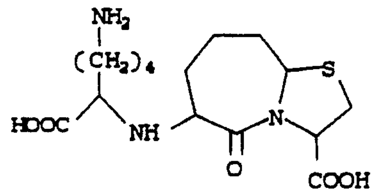

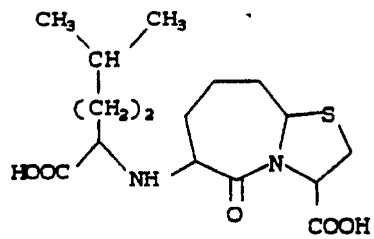

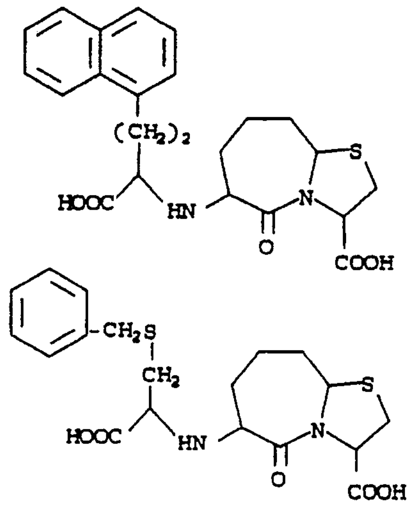

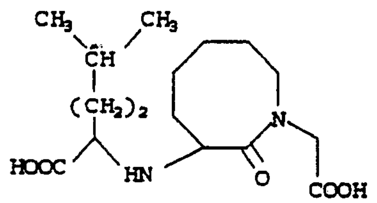

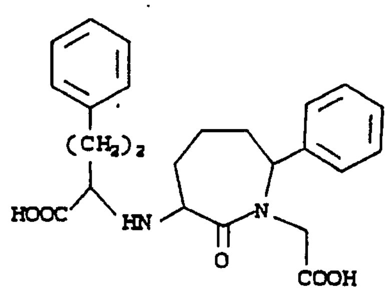

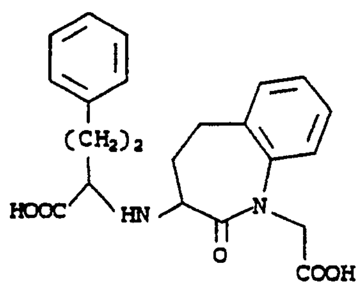

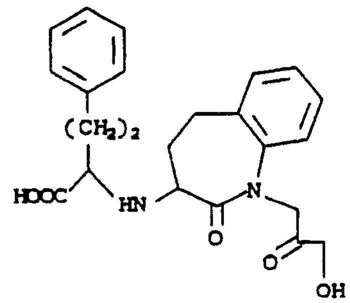

3. The ophthalmic formulation of Claim 1 comprising 0.005 to 2.5% by weight of the ACE inhibitor and 0.005 to 2.5% by weight of a CA inhibitor of formula

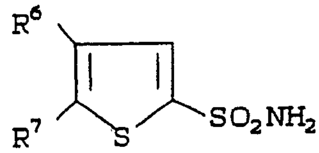

or an ophthalmologically acceptable salt thereof, wherein:
$R^6$ is 1) hydrogen; $R^7$ is

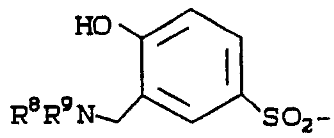

or

$R^6$ and $R^7$ taken together with the carbons to which they are attached represent:

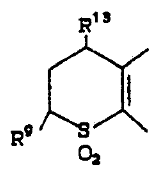

,

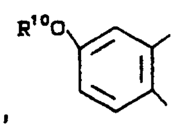

, or

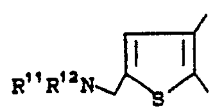

wherein $R^{13}$ is -OH or $-NHR^8$;
$R^8$ and $R^9$ are independently hydrogen or $C_{1-5}$ alkyl;
$R^{10}$ is hydrogen or $C_{2-5}$ alkanoyl;
$R^{11}$ is hydrogen or $C_{1-3}$ alkoxy-$C_{2-4}$alkoxy-$C_{2-4}$alkyl; and
$R^{12}$ is $C_{1-3}$ alkoxy-$C_{2-4}$ alkyl.

4. The ophthalmic formulation of Claim 3 wherein the CA inhibitor is selected from:

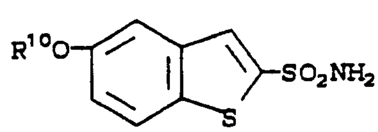

wherein $R^{10}$ is hydrogen or acetyl,

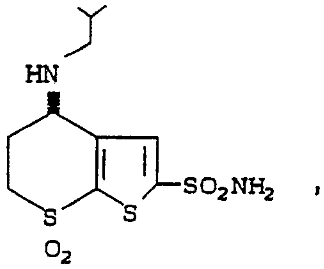

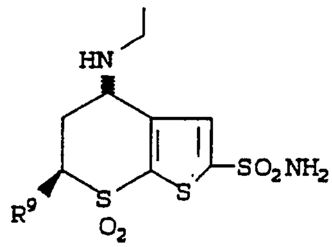

wherein $R^9$ is $-CH_3$ or $-C_2H_5$;

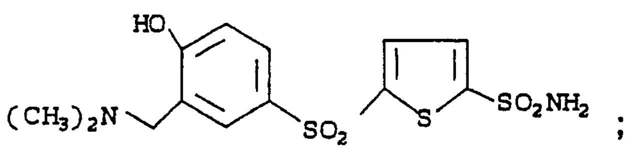

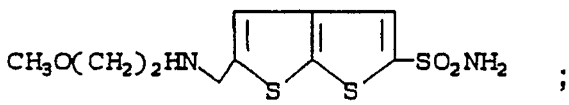

and

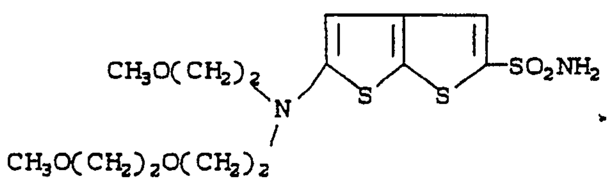

5. The formulation of Claim 2 comprising 0.005 to 2.5% by weight of the ACE inhibitor and 0.005 to 2.5% by weight of a CA inhibitor of formula:

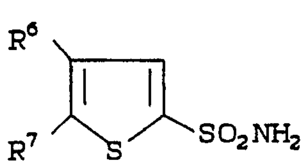

or an ophthalmologically acceptable salt thereof, wherein:
$R^6$ is hydrogen;
$R^7$ is

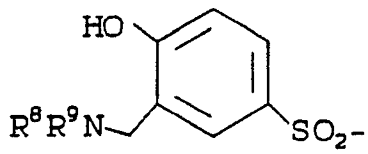 or

$R^6$ and $R^7$ taken together with the carbons to which they are attached represent:

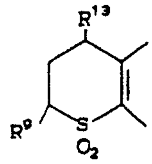 , 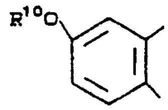 , or 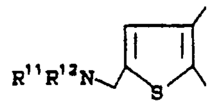

wherein $R^{13}$ is -OH or $-NHR^8$;
$R^8$ and $R^9$ are independently hydrogen or $C_{1-5}$alkyl;
$R^{10}$ is hydrogen or $C_{2-5}$alkanoyl;
$R^{11}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.

6. The use of an ACE inhibitor of formula I for the preparation of a medicament useful for treating glaucoma and ocular hypertension associated therewith:

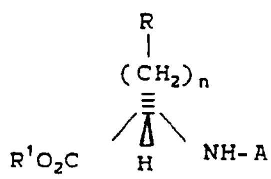

I

or an ophthalmologically acceptable salt thereof, wherein:

R is

1) hydrogen,
2) phenyl,either unsubstituted or substituted with hydroxy, $C_{1-3}$alkoy, $C_{1-3}$alkyl, amino-$C_{1-3}$ alkyl, mono-($C_{1-3}$alkyl)amino-$C_{1-3}$alkyl, or di($C_{1-3}$alkyl)amino-$C_{1-3}$alkyl,
3) 1-naphthyl,
4) 4-piperidinyl,
5) pyridyl,
6) quinolinyl,
7) thiazolyl, or
8) amino either unsubstituted or substituted with one or two $C_{1-3}$alkyl groups; n is 1 to 5;

$R^1$ is

1) H,
2) $C_{1-6}$alkyl; or
3) phenyl-$C_{1-3}$alkyl A is

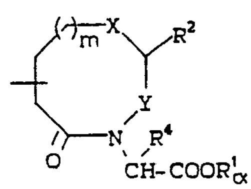

wherein:

m is 0, 1 or 2;
X is
1)

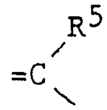 ,

2) $-CH_2-$
3) -S-
4) -SO-, or
5) $-SO_2-$; Y is $CHR^3$ or $N-R^3$;
$R^1{}_\alpha$ is
1) H,
2) $C_{1-6}$alkyl; or
3) phenyl-$C_{1-3}$alkyl; $R^2$ is
1) H,
2) $C_{1-3}$alkyl,
3) 2-thienyl, or
4) phenyl; $R^4$ is
1) hydrogen, or
2) $C_{1-3}$alkyl; $R^3$ and $R^5$ are hydrogen, or
$R^2$ and $R^3$, or $R^2$ and $R^5$ taken together with the atoms to which they are attached, represent:

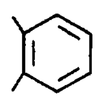

; or
$R^3$ and $R^4$ taken together with the atoms to which they are attached represent:

1) 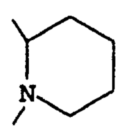 or

2) 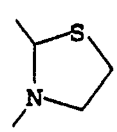

3) 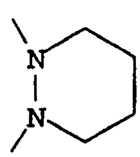 .

7. The use as claimed in Claim 6 wherein the ACE inhibitor is selected from:

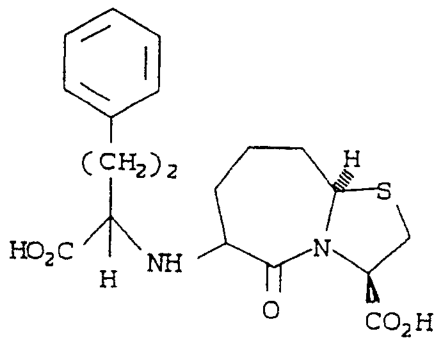

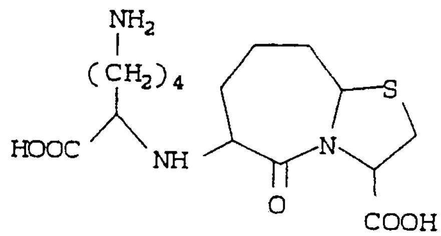

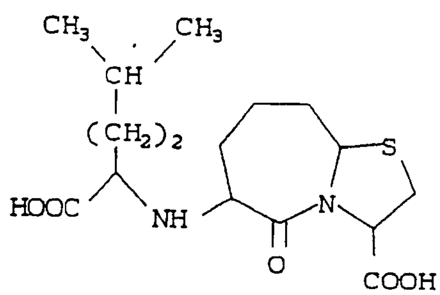

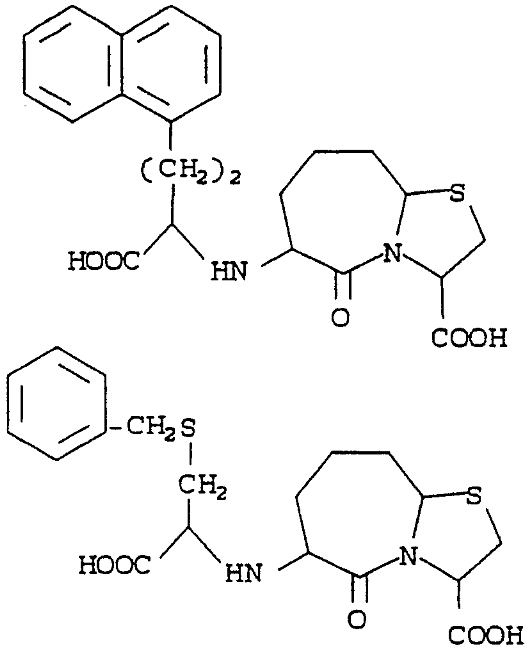

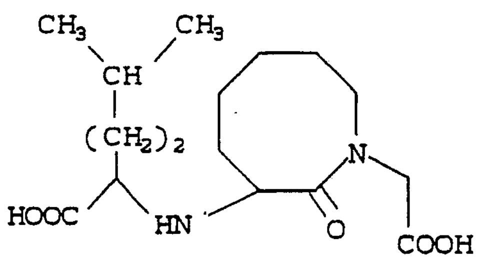

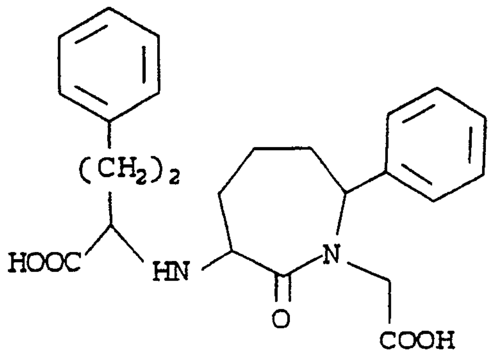

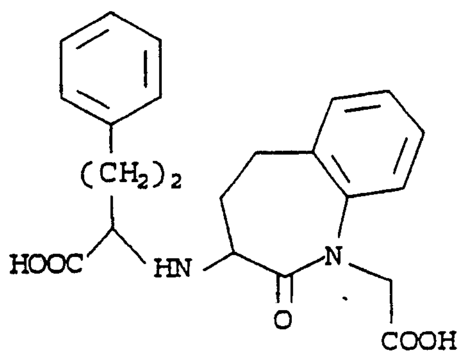

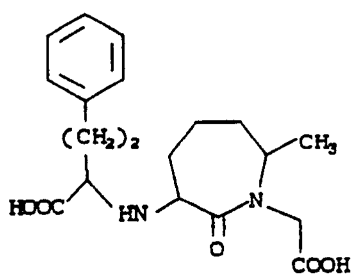

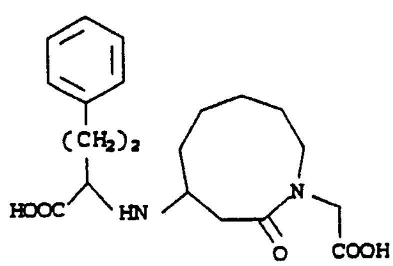

8. The use as claimed in Claim 6 which medicament contains a CA inhibitor of formula

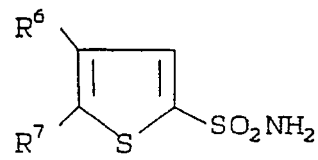

or an ophthalmologically acceptable salt thereof, wherein:
$R^6$ is hydrogen;
$R^7$ is

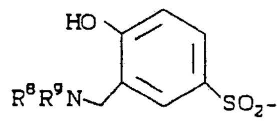

or

$R^6$ and $R^7$ taken together with the carbons to which they are attached represent:

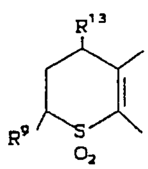

,

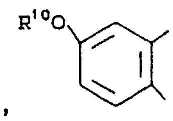

, or

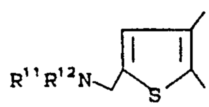

wherein $R^{13}$ is -OH or -$NHR^8$;
$R^8$ and $R^9$ are independently hydrogen or $C_{1-5}$alkyl;
$R^{10}$ is hydrogen or $C_{2-5}$alkanoyl;
$R^{11}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.

9. The use as claimed in Claim 8 wherein the CA inhibitor is selected from:

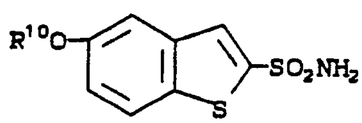

wherein $R^{10}$ is hydrogen or acetyl,

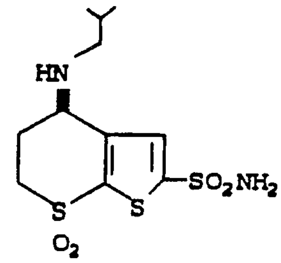 ,

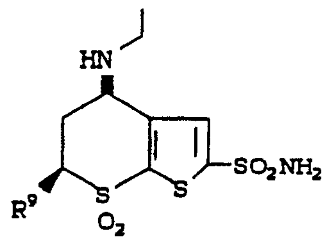

wherein $R^9$ is $-CH_3$ or $-C_2H_5$;

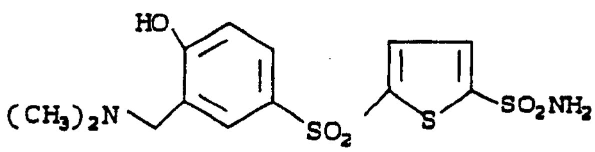 ;

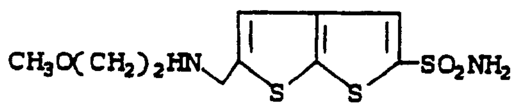 ;

and

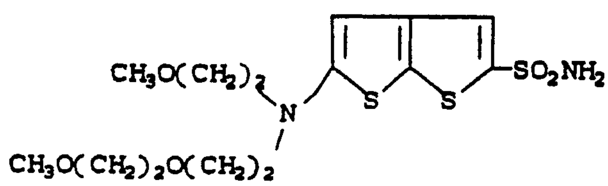

10. The use as claimed in Claim 7 wherein the CA inhibitor has the formula:

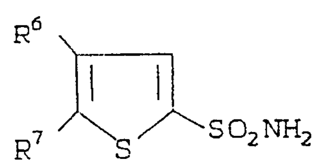

or an ophthalmologically acceptable salt thereof, wherein:
$R^6$ is hydrogen;
$R^7$ is

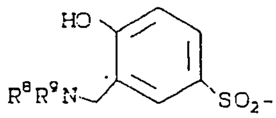

or

$R^6$ and $R^7$ taken together with the carbons to which they are attached represent:

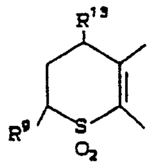

,

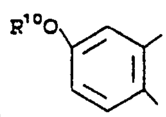

, or

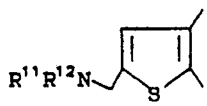

wherein $R^{13}$ is -OH -$NHR^8$;
$R^8$ and $R^9$ are independently hydrogen or $C_{1-5}$alkyl;
$R^{10}$ is hydrogen or $C_{2-5}$alkanoyl;
$R^{11}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.